Europäisches Patentamt

European Patent Office

Office européen des brevets

(19)

(11) Veröffentlichungsnummer: **0 397 011**
**A2**

## (12) EUROPÄISCHE PATENTANMELDUNG

(21) Anmeldenummer: 90108260.2

(51) Int. Cl.5: **A61M 16/01**

(22) Anmeldetag: 30.04.90

(30) Priorität: 10.05.89 US 349829

(43) Veröffentlichungstag der Anmeldung:
**14.11.90 Patentblatt 90/46**

(84) Benannte Vertragsstaaten:
**DE FR GB SE**

(71) Anmelder: **Drägerwerk Aktiengesellschaft**
**Moislinger Allee 53-55**
**D-2400 Lübeck 1(DE)**

(72) Erfinder: **Jaklitsch, Roman R., Dr.**

**Wakenitzmauer 21**
**D-2400 Lübeck(DE)**
Erfinder: **Wallroth, Carl F., Dr.**
**Stresemannstrasse 1**
**D-2400 Lübeck(DE)**
Erfinder: **Loughlin, Patrick J.**
**2105 E. Shelby Street**
**Seattle, WA 98112(US)**
Erfinder: **Westenskow, Dwayne D.**
**3439 Winesap Road**
**Salt Lake City, Utah 84121(US)**

(54) **Anästhesiebeatmungsgerät mit Atemkreislauf und Regelkreisen für Anästhesiegasbestandteile.**

(57) Ein Anästhesiebeatmungsgerät mit Atemkreisführung und Regelkreisen zur Beeinflussung der Anästhesiegase soll derart verbessert werden, daß eine geänderte Sollwertvorgabe eines Anästhesiegasbestandteils in zeitoptimierter Weise einstellbar und der zugehörige Regler des Regelkreises an die Systemparameter des Atemkreislaufs selbsttätig adaptierbar ist. Hierzu ist vorgesehen, den Regelkreis mit einem Trennschalter (311) zu versehen, der bei Einstellung eines neuen Sollwertes (814) während einer vorbestimmten Zeitdauer in Öffnungsstellung schaltet und danach die Stellgröße an der Dosiereinheit (307) auf einen vom Sollwert abweichenden Anflutungswert eingestellt wird.

Aus dem zeitlichen Verlauf der Konzentration des Anästhesiegasbestandteils werden Einstellparameter für den zugehörigen Regler (302) errechnet.

FIG. I

Die Erfindung betrifft ein Anästhesiebeatmungsgerät mit einem Atemkreislauf, in welchen die für die Beatmung notwendigen Anästhesiegase zudosierbar und über Regelkreise beeinflußbar sind. Die Erfindung betrifft ebenfalls ein Verfahren zum Betreiben eines Anästhesiebeatmungsgerätes.

Ein bekanntes Anästhesiebeatmungsgerät mit geschlossenem Atemkreislauf aus der EP 121 255 benutzt für die Zudosierung von Anästhesiegas einen aufwendigen Regelkreis, in welchem die Anästhesiegasbestandteile durch entsprechende Sensoren abgefühlt und die Sensorsignale zur Ansteuerung einer Dosiereinheit benutzt werden. Es wird zu jeder Zeit während einer Beatmung der Füllzustand des Atemgases im Atemkreislauf bestimmt und die erforderliche Frischgasmenge zugeführt.

Der vorhandene Regelkreis für die Anästhesiegasdosierung ist im wesentlichen zur Aufrechterhaltung eines quasistationären Betriebszustandes konzipiert; d. h. es werden nach einem festgelegten, unveränderbaren Regelalgorithmus die zuvor eingestellten Konzentrationswerte der Anästhesiegasbestandteile gehalten und nur soviel Gas zugeführt wie verbraucht wurde.

Nachteilig bei dem bekannten Anästhesiebeatmungsgerät ist, daß bei Veränderung des Konzentrationsanteils einzelner Anästhesiegasbestandteile, beispielsweise der Anästhesiemittelkonzentration, die neuen Werte erst nach z. T. erheblichen Einstellzeiten erreicht werden, wobei sich die gemessene Konzentration im Atemkreislauf asymptotisch dem neuen Sollwert nähert. Dies ist für eine Vielzahl der Anwendungsfälle nicht tolerierbar; z. B. beim Übergang von der Einleitungsphase mit hoher Anästhesiemittelkonzentration auf die Erhaltungsphase mit geringer Konzentration.

Ein Anästhesiebeatmungsgerät mit gesteuerter Zufuhr des Anästhesiemitteldampfes ist in der GB-Z: Br. J. Anaesth. (1983), 55, 1065-1075 beschrieben. Das den Patienten mit Atemgas versorgende Atemsystem ist an einen Ventilator zur maschinellen Beatmung angeschlossen und wird über eine Gasdosiereinheit mit den Anästhesiegasen Lachgas, Sauerstoff und Anästhesiemittel versorgt. Eine zentrale, mikroprozessorgesteuerte Steuereinheit beeinflußt einerseits als Sollwertgeber die Anästhesiemittel-Dosiereinheit und registriert andererseits den Istwert, der mit einem Anästhesiemittel-Sensor im Atemsystem stromabwärts vom Patienten gemessen wird. Bei Einstellung eines neuen Anästhesiemittel-Sollwertes gibt die Steuereinheit zunächst an die Dosiereinheit ein Stellgrößensignal, das z.B. um ein Vielfaches über dem einzustellenden neuen Sollwert liegt. Diese erste, etwa 9 Atemzüge dauernde Phase, dient dazu, aus der sich spontan einstellenden Anästhesiemittel-Konzentrationsänderung im Atemsystem systemspezifische Parameter zu ermitteln.

Diese Parameter und weitere in der Steuereinheit gespeicherte Konstanten werden miteinander verknüpft, um während einer zweiten Phase, die etwa 90 Atemzüge dauert, die Anästhesiemittelkonzentration im Atemsystem schrittweise dem neuen Sollwert anzunähern. In der dritten Phase wird die Anästhesiemittelkonzentration durch Zuschalten eines Reglers auf den gewählten Sollwert eingestellt.

Nachteilig bei diesem Anästhesiebeatmungsgerät ist, daß ein neuer Sollwert für die Anästhesiemittelkonzentration erst nach einem aufwendigen Meß- und Rechenprogramm einstellbar ist, wobei Konstanten aus Tabellen, die zuvor in die Steuereinheit eingegeben werden müssen, zu berücksichtigen sind. Die Aufteilung in drei Phasen ist für die klinische Routine nicht praktikabel. Es besteht zudem keine direkte Verkopplung zwischen den in der ersten Phase gemessenen Parametern und den Einstellwerten des Anästhesiemittel-Reglers, der in der dritten Phase zugeschaltet wird.

Die Erfindung geht somit von der Aufgabe aus, ein Anästhesiebeatmungsgerät der genannten Art so zu verbessern, daß eine geänderte Sollwertvorgabe eines Anästhesiegasbestandteils in zeitoptimierter Weise einstellbar und der dazugehörige Regler des Regelkreises an die Systemparameter des Atemkreislaufs selbsttätig adaptierbar ist. Außerdem soll ein Verfahren zum Betreiben eines Anästhesiebeatmungsgerätes angegeben werden.

Zur Lösung der Aufgabe ist vorgesehen, daß mindestens ein Anästhesiegas-Regelkreis mit einem Trennschalter versehen ist, wobei zur Einstellung eines neuen Anästhesiegas-Sollwertes S1 die Steuereinheit

- den Trennschalter während einer vorbestimmten Zeitdauer in Öffnungsstellung schaltet,
- die Anästhesiegas-Stellgröße an der Dosiereinheit sprunghaft auf einen vom Sollwert S1 abweichenden Anflutungssollwert S2 einstellt,
- aus dem mit der Meßeinrichtung in mindestens einem der Zweige gemessenen zeitlichen Verlauf der Anästhesiegaskonzentration Atemkreissystemparameter $T_1$ und $T_2$ bestimmt und hieraus zumindest Einstellparameter ($K$, $C_I$, $C_D$) für den Anästhesiegas-Regler errechnet; und daß die Steuereinheit

- bei Erreichen des Anästhesiegaskonzentrationswertes S3,
- den Trennschalter in Schließstellung schaltet, wodurch der Anästhesiegas-Regler die Dosiereinheit zur Einstellung des Sollwertes S1 beeinflußt.

Der Vorteil der Erfindung liegt im wesentlichen darin, daß der Regelkreis für eine bestimmte Zeitspanne aufgetrennt und der Atemkreislauf mit dem zu ändernden Anästhesiegas geflutet werden kann. Erst bei Erreichen eines Konzentrationswertes nahe dem einzustellenden neuen Sollwert wird der Regelkreis wieder geschlossen, wodurch der Regler die Dosiereinheit des betreffenden Anästhesiegases zur Einstellung des neuen Sollwertes beeinflußt. Außerdem sollen aus dem zeitlichen Verlauf der Anästhesiegaskonzentration Atemkreis-Systemparameter bestimmt und hieraus Einstellparameter für den Anästhesiemittel-Regler errechnet werden. Der zeitliche Verlauf der Anästhesiegaskonzentration im Atemkreislauf ist beispielsweise abhängig von der zuströmenden Gasmenge des Anästhesiegasbestandteils, dem Atemkreisvolumen und der Gaszirkulation innerhalb des Atemkreises. Ein Regler mit einer festen Einstellung kann nur für eine bestimmte Parameterkonstellation optimale Ergebnisse liefern. Durch eine bedarfsadaptierte Anpassung der Einstellparameter, die aus dem zeitlichen Konzentrationsverlauf errechnet werden, liefert der Regler zeitoptimierte Einstellwerte speziell auch dann, wenn zwischenzeitlich Komponenten des Atemkreislaufs - z. B. durch Hinzufügen von Schläuchen zwischen Patient und Anästhesiebeatmungsgerät -verändert wurden. Durch die Messung des zeitlichen Verlaufs der Konzentrationsänderung sind derartige Modifikationen feststellbar.

Es ist vorteilhaft, als Anästhesiegasbestandteil die Anästhesiemittelkonzentration zu verwenden, da diese, durch das routinemäßige Narkosefahren bedingt, ohnehin verändert werden muß. So wird z. B. während der Einleitungsphase mit hoher Anästhesiemittelkonzentration, in der Erhaltungsphase mit geringer Anästhesiemittelkonzentration in dem zuströmenden Anästhesiegas gearbeitet. Während der Ausleitungsphase am Ende der Narkose wird die Anästhesiemittelkonzentration vollständig auf den Wert Null zurückgefahren. Als weiterer Anästhesiegasbestandteil bietet sich die Sauerstoffkonzentration im Atemkreislauf an. Sie ist veränderbar, indem der Atemkreislauf mit Sauerstoff oder Lachgas geflutet wird. Hierbei darf die für die Beatmung notwendige Mindeskonzentration an Sauerstoff jedoch nicht unterschritten werden.

Es ist zweckmäßig, bei einer Konzentrationsänderung die Stellgröße auf ein Vielfaches des Sollwertes - den Anflutungssollwert S2 - einzustellen. Dieser Faktor kann bei einer Konzentrationserhöhung bis zum 10fachen des Sollwertes, bei einer Konzentrationserniedrigung eine völlige Zurücknahme der Stellgröße auf den Wert Null zur Folge haben.

Nach Erreichen des 0,9fachen vom Sollwert S1 wird der Regelkreis bei einer Konzentrationserhöhung geschlossen und der Regler beeinflußt die Stellgröße. Die Anästhesiegaskonzentration ist nun auf den zuvor eingestellten Sollwert einregelbar. Bei einer Konzentrationserniedrigung wird der Regler bei dem 1,1fachen des neuen Sollwertes S11 eingeschaltet.

Es ist zweckmäßig, die Systemparameter des Atemkreises aus dem zeitlichen Verlauf der betreffenden Anästhesiegaskonzentration zu bestimmen. Diese Systemparameter ergeben sich im wesentlichen aus der Spülzeit des Atemkreises mit dem geänderten Anästhesiegasparameter und sind beeinflußbar durch den Anästhesiegasstrom, das Atemhubvolumen, die Atemfrequenz und die Anästhesiegasaufnahme durch den Patienten.

Es ist vorteilhaft, neben der Anästhesiemittelkonzentration auch Regelkreise für Sauerstoffkonzentration, Atemkreisvolumen und Kohlendioxidkonzentration vorzusehen und diese Regelkreise über Koppelglieder verbindbar zu machen.

Diese Koppelglieder sind geeignet, um abhängige Größen regelbar zu machen. Wird beispielsweise der Anästhesiegasstrom erhöht, muß, um im Atemkreislauf eine konstante Anästhesiemittelkonzentration zu bewahren, die Anästhesiemitteldampfmenge ebenfalls nachgeführt werden; d.h. die Anästhesiemittel-Dosierpumpe muß eine höhere Flüssigkeitsmenge dem Anästhesiegasstrom zuführen. Weiter ist es vorteilhaft, die Empfindlichkeit einzelner Regler in Abhängigkeit von bestimmten Anästhesiegasparametern zu steuern. So ist beispielsweise der Atemkreisvolumen-Regler bei einem geschlossenen Atemkreislauf die bestimmende Komponente für die Anästhesiegaszufuhr in den Atemkreislauf, da nur das Volumen zugeführt wird, was verbraucht wurde. Wird hingegen eine Anästhesiegasmenge dosiert, die um ein Vielfaches über dem Verbrauch liegt, wie es beim halbgeschlossenen Atemkreislauf der Fall ist, ist der Atemkreisvolumen-Regler von sekundärer Bedeutung, da das überschüssige Anästhesiegas nach jedem Atemzug über ein Überschußgas-Ablaßventil in die Umgebung entlassen wird. Eine Empfindlichkeitsanpassung ist ebenfalls bei dem Kohlendioxid-Regler vorgesehen. Der Verstärkungsfaktor wird in Abhängigkeit vom Atemkreisvolumen und dem Atemminutenvolumen eingestellt. Bei großem Atemminutenvolumen, d.h. starker Ventilation des Patienten, ist die Empfindlichkeit maximal, und der Kohlendioxid-Regler reagiert unverzüglich auf eine im Atemkreislauf gemessene Kohlendioxid-Konzentrationsänderung.

Ein weiteres Koppelglied ist die Anpaßschaltung für den Anästhesiemittel-Regler, die diesem die Einstellparameter einprägt, wobei die Vorgaben von der Steuereinheit geliefert werden.

Durch die Verkoppelung der Regelkreise soll erreicht werden, daß in Abhängigkeit von den eingestellten Beatmungsparametern das Anästhesiebeatmungsgerät selbsttätig die vorteilhafteste Reglerkonstellation auswählt.

Es ist zweckmäßig, einzelne Koppelglieder und einzelne Regler von der Steuereinheit ansteuerbar zu machen. So können beispielsweise die Einstellparameter des Anästhesiemittel-Reglers von der Steuereinheit errechnet und über eine Anpaßschaltung übertragen werden. Weiter ist vorstellbar, daß weitere Parameter in die Berechnung der Regler-Einstellparameter einbezogen werden, so z.B. patientenbezogene Größen wie Körpergewicht, Körpergröße usw. Dieses kann erforderlich sein, wenn Konzentrationsänderungen nur beschränkt durchführbar sind, z.B. bei Kleinkindern.

Es ist vorteilhaft, die Reihenfolge der Sollwertvorgaben durch die Steuereinheit zu steuern. Sollen beispielsweise mehrere Anästhesiegasbestandteile gleichzeitig geändert werden, können durch die Steuereinheit Prioritäten für die Sollwertvorgaben festgelegt werden. Eine zweckmäßige Prioritätenfolge wäre beispielsweise, durch die Zufuhr von Sauerstoff zunächst die Sauerstoff-Konzentration im Atemkreislauf einzustellen. Mit zweiter Prioritätsstufe wird die Anästhesiemittelkonzentration eingestellt für die Anästhesietiefe. Durch Beeinflussung der Atemfrequenz am Ventilator ist dann die Kohlendioxidkonzentration einregelbar. Der Volumenregelkreis schließlich wird aktiviert, wenn alle anderen Regelkreise eingeschwungen sind, um das Atemkreisvolumen auf den vorgewählten Wert einzustellen.

Weitere Einzelheiten der Erfindung werden anhand eines in der Zeichnung dargestellten Ausführunsbeispiels erläutert.

Es zeigen

Fig. 1 einen Atemkreislauf mit Regelkreisen für Anästhesiegasbestandteile,

Fig. 2 eine Sprungantwort bei Änderung der Anästhesiemittelkonzentration.

Fig. 3 die Ermittlung der Reglerparameter K, CI, CD aus T1 und T2.

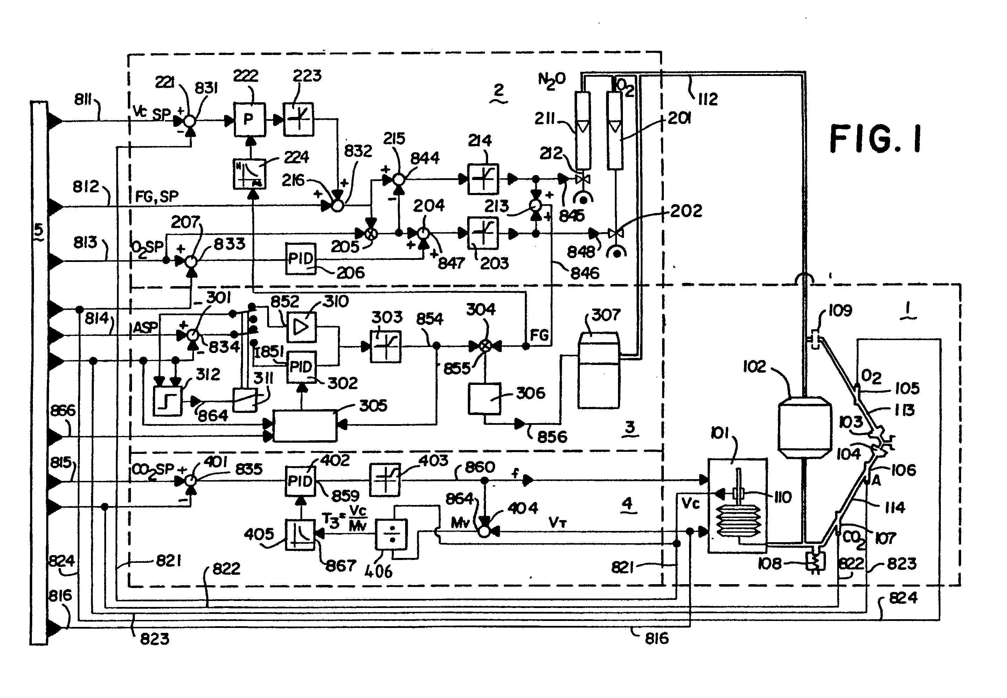

Das in Fig. 1 dargestellte Anästhesiebeatmungsgerät besteht aus dem Atemkreislauf (1), der Gasdosiereinheit (2) für die Anästhesiegasbestandteile Sauerstoff und Lachgas, der Anästhesiemittel-Dosiereinheit (3), dem Kohlendioxid-Regelkreis (4) und der Steuereinheit (5) mit integriertem Rechenteil. Die verschiedenen Bestandteile sind mit einer gestrichelten Linie umrandet.

Der Atemkreislauf (1) mit einem Einatemzweig (113) und einem Ausatemzweig (114) sorgt für die Beförderung des Atemgases zu einem nicht dargestellten Patienten. Er wird gebildet aus dem Ventilator (101), der vorzugsweise als Kolben-Zylinder-Einheit ausgeführt ist, dem Kohlendioxidabsorber (102) zur Entfernung des vom Patienten ausgeatmeten Kohlendioxids, Richtungsventilen (103, 104) zur Steuerung der Atemgasrichtung, Sensoren (105, 106, 107) zum Analysieren der Anästhesiegasbestandteile und einem Überschußgas-Ablaßventil (108). Ferner ist vorgesehen, bedarfsweise ein Kohlefilter (109) in den Atemkreislauf (1) einzuschalten, um das von der Anästhesiemittel-Dosiereinheit (3) gelieferte Anästhesiemittel teilweise oder auch vollständig entfernen zu können. Dieses kann notwendig sein, wenn die Anästhesiemittelkonzentration kurzfristig reduziert werden muß, z. B. beim Übergang von der Einleitungsphase in die Erhaltungsphase der Narkose. Der Atemkreislauf (1) ist für den Betrieb mit teilweiser oder auch vollständiger Rückatmung ausgelegt, wodurch die Anästhesiegasbestandteile zu einem hohen Grade ausgenutzt werden. Bei vollständiger Rückatmung muß nur der Gasverlust durch Verbrauch und Leckagen wieder zugeführt werden.

Zur Analyse der Anästhesiegasbestandteile sind ein Sauerstoffsensor $O_2$ (105) auf der Inspirationsseite und ein Anästhesiemittelsensor (106) und ein Kohlendioxidsensor $CO_2$ (107) auf der Exspirationsseite vorgesehen.

Über den Anästhesiegas-Anschluß (111) strömt Anästhesiegas aus der Gasdosiereinheit (2) und der Anästhesiemittel-Dosiereinheit (3) in den Atemkreislauf (1). Überschüssiges Gas kann über das Überschußgas-Ablaßventil (108) entfernt werden.

Die Gasdosiereinheit (2) dient zur Dosierung der Anästhesiegase Sauerstoff und Lachgas, deren Einzeldurchflußmengen über Durchflußmeßröhren (201, 211) angezeigt werden und über elektromotorisch betriebene Stellventile (202, 212) einstellbar sind. Die über die Signalanschlüsse (848, 845) zugeführten Stellgrößen zur Ansteuerung der Stellventile (202, 212) werden von dem Sauerstoffkonzentrations-Regler (206) und dem Atemkreisvolumen-Regler (222) geliefert.

Die Anästhesiemitteldosiereinrichtung (3) besteht im wesentlichen aus der Anästhesiemittel-Dosierpumpe (307), die flüssiges Anästhesiemittel in die Anästhesiegas-Leitung (112) dosiert, wo es verdampft und sich mit den Anästhesiegasen Sauerstoff und Lachgas vermischt. Alternativ ist vorstellbar, daß das Anästhesiemittel direkt mit einer elektrisch betriebenen Kolbenspritze in den Atemkreislauf (1) eingespritzt wird und dort verdampft. Die Ansteuerung der Anästhesiemittel-Dosierpumpe (307) erfolgt über den Signalanschluß (856), der mit dem Anästhesiemittel-Regler (302) und dem Verstärker (310) verbunden ist.

Der Kohlendioxid-Regelkreis (4) besteht aus dem Kohlendioxid-Regler (402), dem Kohlendioxidsensor (107) als Istwertgeber und dem Füllstandsdetektor (110) für die Atemgasmenge im Atemkreislauf 1. Das Stellgrößensignal wird über den Anschluß (860) in Form der Beatmungsfrequenz dem Ventilator (101) zugeführt. Durch Veränderung der Beatmungsfrequenz wird die Kohlendioxidkonzentration im Atemkreislauf (1) eingestellt.

Die Steuereinheit (5) mit integriertem Rechenteil ist Sollwertgeber für die Gasdosiereinheit (2), die Anästhesiemittel-Dosiereinheit (3), den Kohlendioxid-Regelkreis (4) und das Atemhubvolumen (816) des Ventilators (101). Angeschlossen sind die Signalleitungen für Atemkreisvolumen-Sollwert Vc SP (811), Anästhesiegas-Sollwert FG SP (812), Sauerstoffkonzentrations-Sollwert $O_2$ SP (813), Anästhesiemittelkonzentrations-Sollwert A SP (814), Kohlendioxidkonzentrations-Sollwert $CO_2$ SP (815) und Atemhubvolumen-Sollwert Vc SP (816).

Zum Betrieb sei zunächst ein geschlossener Atemkreislauf (1) angenommen; d. h. es wird soviel Anästhesiegas zugeführt wie verbraucht wird und durch Leckagen verloren geht.

An der Steuereinheit (5) sei ein Sauerstoffkonzentrations-Sollwert (813) und ein Atemkreisvolumen-Sollwert (811) vorgegeben. Der Anästhesiegas-Sollwert (812) ist beim geschlossenen Atemkreislauf (1) auf Null gesetzt, da die zuzuführende Anästhesiegasmenge allein aus der für das geschlossene System geltenden Bedingung bestimmt wird, daß die Sauerstoffkonzentration und das Atemkreisvolumen im stationären Fall konstant zu halten sind. Mit dem Sauerstoffsensor (105) wird der Istwert im Atemkreislauf gemessen und über die Signalleitung (824) der Subtraktionsstelle (207) zugeführt. Der Ausgang (833) ist an den Sauerstoffkonzentrations-Regler (206) angeschlossen. Der Füllstandsdetektor (110) liefert den Istwert für das Atemkreisvolumen Vc und führt diesen über die Signalleitung (821) an die Subtraktionsstelle (221). Der Ausgang (831) ist an den Atemkreisvolumen-Regler (222) angeschlossen.

Das Steuersignal für die Anästhesiegasmenge, die pro Zeiteinheit in den Atemkreislauf (1) zudosiert werden muß, um das Atemkreisvolumen auf dem Sollwert (811) zu halten, entsteht am Ausgang (832) der Additionsstelle (216).

Über die Multiplikationsstelle (205), die Additionsstelle (204) und die Subtraktionsstelle (215) werden aus dem Steuersignal am Ausgang (832) und dem Sauerstoffkonzentrations-Sollwert (813) anästhesiegasspezifische Stellsignale gebildet, die von den Ausgängen (844, 847) den Signalanschlüssen (848, 845) der Stellventile (202, 212) zugeführt werden. Die Summe der an den Durchflußmeßröhren (211, 201) gemessenen Teilgasströme Sauerstoff $O_2$ und Lachgas $N_2O$ ist proportional dem Steuersignal am Ausgang (832). Wird es erhöht, steigen in gleichem Maße die Gasflüsse an den Durchflußmeßröhren (211, 201). Wird hingegen der Sauerstoffkonzentrations-Sollwert (813) verändert, z.B. erhöht, führt dies am Ausgang (844) der Subtraktionsstelle (215) zu einem entsprechend kleineren Steuersignal; am Ausgang (847) der Additionsstelle (204) erhöht sich das Steuersignal. Das Lachgas-Stellventil (212) wird in dem Maße geschlossen, wie das Sauerstoff-Stellventil (202) geöffnet wird. Die Summe der an den Durchflußmeßröhren (211, 201) gemessenen Teilgasströme hingegen bleibt konstant. Die Begrenzer (203,214) begrenzen die Signalspannungen an den Ausgängen (844,847) auf einen oberen und unteren Grenzwert, der proportional zum minimal und maximal dosierbaren Sauerstoff- bzw. Lachgas-Gasfluß ist.

Der Atemkreisvolumen-Regler (222) ist als Proportionalregler ausgeführt, dessen Verstärkung über den Kennliniengeber (224) in Abhängigkeit vom Anästhesiegasfluß-Signal (846) veränderbar ist. Im geschlossenen Atemkreislauf (1), wo nur das verbrauchte Anästhesiegas substituiert wird, ist die Verstärkung P des Atemkreisvolumen-Reglers (222) und damit die Empfindlichkeit des Regelkreises maximal. Der Atemkreisvolumen-Regelkreis ist die bestimmende Komponente für die Anästhesiegasdosierung in den Atemkreislauf (1). Ist hingegen der Anästhesiegas-Sollwert (812) hoch eingestellt - wie beispielsweise beim halbgeschlossenen System - tritt der Atemkreisvolumen-Regelkreis in den Hintergrund, da stets genügend Anästhesiegas dem Atemkreislauf (1) zugeführt wird. Der Begrenzer (223) begrenzt das Ausgangssignal (841) auf den oberen und unteren Wert für das Atemkreisvolumen.

Die Anästhesiemittel-Dosiereinheit (3) dient dazu, eine vorgewählte Anästhesiemittelkonzentration einzustellen. Der Anästhesiemittelkonzentrations-Sollwert (814) wird an der Subtraktionsstelle (301) mit dem vom Anästhesiemittelsensor (106) gemessenen Istwert verglichen. Die den Istwert führende Signalleitung (823) und der Anästhesiemittelkonzentrations-Sollwert (814) sind an einen Grenzwertschalter (312) angeschlossen.

Im folgenden soll die Funktion des Grenzwertschalters (312) beschrieben werden.

Überschreitet das Differenzsignal zwischen Sollwert (814) und dem über die Signalleitung (823) übertragenen Istwert einen vorgebbaren oberen Grenzwert, liefert der Grenzwertschalter (312) einen Steuerpuls über die Signalleitung (864) an den Trennschalter (311), der in Öffnungsstellung umschaltet.

Der Eingang (852) des Verstärkers (310) ist mit dem Anästhesiemittel-Sollwert (814) verbunden. Ist der Verstärkungsfaktor des Verstärkers (310) beispielsweise auf den Faktor 10 eingestellt, wird die 10-fache Menge Anästhesiemittel in den Atemkreislauf (1) dosiert.

Unterschreitet das Differenzsignal am Grenzwertschalter (312) einen unteren vorgebbaren Grenz-Wert - d. h. der neue Sollwert im Atemkreislauf (1) ist nahezu erreicht - schaltet der Trennschalter (311) in Schließstellung, und der Eingang (851) des Anästhesiemittel-Reglers (302) ist mit dem Ausgang (834) verbunden. Der Anästhesiemittel-Regler (302) liefert damit die Stellgröße (854) für die Narkosemittel-Dosiereinheit (307).

Der Begrenzer (303) limitiert die Stellgröße (854) auf die maximal einstellbare Konzentration, z.

B. auf 6 Vol-%. Das Steuersignal (856) für die Narkosemittel-Dosiereinheit (307) für die in den Atemkreislauf (1) abzuführende Anästhesiemittelmenge wird gebildet aus dem an der Multiplikationsstelle (304) gebildeten Produkt (855) aus Stellgröße (854) und Anästhesiegasfluß FG (846). Das Produkt (855), beaufschlagt mit der Dosierrate (306), ergibt das Steuersignal (856) für die Anästhesiemittel-Flüssigkeitsmenge, die von der Anästhesiemittel-Dosiereinheit (307) in die Anästhesiegas-Leitung (112) zu dosieren ist. Der Anästhesiemitteldampf wird zusammen mit dem Anästhesiegas über die Anästhesiegas-Leitung (112) und den Anästhesiegas-Anschluß (111) dem Atemkreislauf (1) zugeführt.

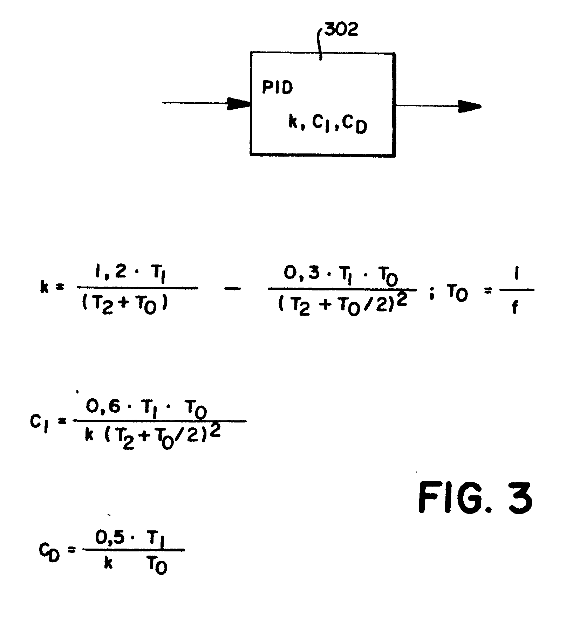

Über die Anpaßschaltung (305) sind die Einstellparameter des Anästhesiemittel-Reglers (302) beeinflußbar. Die Anpaßschaltung (305) ist über eine Signalleitung (866) an die Steuereinheit (5) angeschlossen und erhält von hier die Vorgaben für die Regler-Einstellparameter (Fig. 3). Bei der Berechnung der Regler-Einstellparameter können auch patientenbezogene Parameter berücksichtigt werden, die zuvor in die Steuereinheit (5) eingegeben werden müssen.

Mit der Kombination von Grenzwertschalter (312) und Trennschalter (311) soll erreicht werden, daß bei Änderung des Anästhesiemittel-Sollwertes (814) der Anästhesiemittel-Regler (302) für eine gewisse Zeit ausgeschaltet und der Atemkreislauf (1) mit einer vom Sollwert abweichenden Anästhesiemittelkonzentration angeflutet und gespült wird. Erst wenn die mit dem Anästhesiemittel-Sensor (106) gemessene Anästhesiemittelkonzentration den Sollwert (814) nahezu erreicht hat, wird der Anästhesiemittel-Regler (302) wieder eingeschaltet, um die Anästhesiemittelkonzentration auf den genauen Wert einzuregeln.

Der Kohlendioxid-Regelkreis (4) dient dazu, eine bestimmte endexspiratorische Kohlendioxidkonzentration einzustellen. Dieser Parameter ist im wesentlichen beeinflußbar durch die Art der Beatmung, beispielsweise der Beatmungsfrequenz am Ventilator (101). An der Subtraktionsstelle (401) werden der Kohlendioxid-Sollwert $CO_2$ SP (815) und das von der Signalleitung (822) übertragene Istwertsignal zusammengeführt. Das Istwertsignal wird von dem Kohlendioxidsensor (107) geliefert. Die Regelabweichung am Ausgang (835) wird dem Kohlendioxid-Regler (402) zugeführt. Das Signal am Ausgang (859) wird über einen Begrenzer (403) geführt, der den Variationsbereich der Stellgröße (860) auf Werte begrenzt, die vom Ventilator (101) verarbeitbar ist. Die Stellgröße (860) ist die Beatmungsfrequenz f des Ventilators (101), d. h. die Anzahl der Hübe pro Minute. Über den Kennliniengeber (405) ist der Verstärkungsfaktor P des Kohlendioxid-Reglers (402) einstellbar. Das Ansteuersignal (867) für den Kennliniengeber (405) setzt sich zusammen aus dem an der Multiplikationsstelle (404) gebildeten Atemminutenvolumen-Signal Mv (864) und dem Atemkreisvolumen-Signal Vc (821). Der Quotient aus Atemkreisvolumen-Signal Vc (821) und Atemminutenvolumen-Signal Mv (864) ergibt das Ansteuersignal $T_3$ 867) für den Kennliniengeber (405). Bei größerem Atemminutenvolumen-Signal (864) ist der Verstärkungsfaktor und damit die Empfindlichkeit maximal.

Das Steuersignal für das Atemhubvolumen Vr (816) wird direkt dem Ventilator (101) zugeführt.

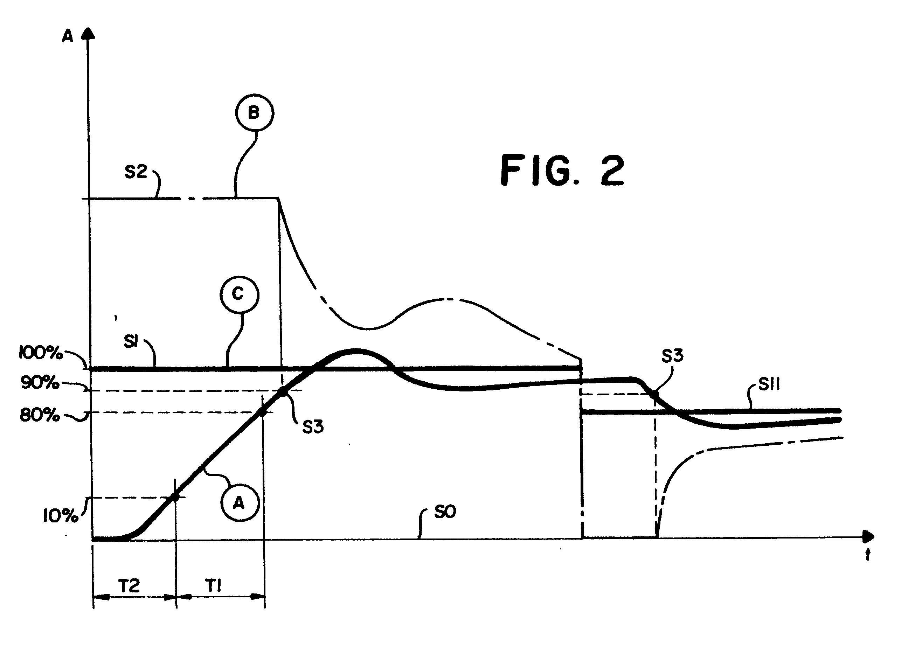

Ein Ausführungsbeispiel für eine Sollwertänderung der Anästhesiemittelkonzentration von S0 auf S1 ist in Fig. 2 dargestellt. Dabei gibt die Kurve (A) die mit dem Anästhesiemittel-Sensor (106) gemessene Konzentration im Atemkreislauf wieder, Kurve (B) ist die Anästhesiemittelkonzentration in der Anästhesiegas-Leitung (112) und Kurve (C) der neu einzustellende Sollwert S1 bzw. S11.

An der Steuereinheit (5) sei eine Änderung des Anästhesiemittel-Sollwertes (814) von S0 auf S1 vorgegeben. Der Grenzwertschalter (312) registriert eine Differenz zwischen dem mit dem Anästhesiemittelsensor (106) gemessenen Istwert und dem neuen Sollwert S1. Da die Differenz über dem voreingestellten Grenzwert liegt, wird über den Ausgang (864) ein Steuerpuls an den Trennschalter (311) gelegt, der diesen in Öffnungsstellung umschaltet, wie in Fig. 1 dargestellt. Der Anästhesiemittel-Sollwert (814) ist mit dem Eingang (852) des Verstärkers (310) verbunden. Durch den Verstärker wird eine um den Verstärkungsfaktor erhöhte Dosierung in Form eines Anflutungssollwertes S2 an der Anästhesiemittel-Dosierpumpe (307) eingestellt. Ist der neue Sollwert S1 beispielsweise 0,5 Vol-% und der Verstärkungsfaktor 10, wird als Anflutungssollwert S2 5 Vol-% Anästhesiemittel in den Atemkreislauf (1) dosiert.

Aus der Sprungantwort der mit dem Anästhesiemittelsensor (106) gemessenen Anästhesiemittelkonzentration werden die Systemparameter T2 und T1 bestimmt (Kurve A). Bei der Sollwertveränderung von S0 auf S1 wird durch einen Zeitgeber in der Steuereinheit (5) der Zeitpunkt Null festgesetzt. Die mit dem Anästhesiemittelsensor (106) gemessene Konzentrationsänderung wird fortlaufend beginnend vom Startpunkt von der Steuereinheit (5) registriert. Der Systemparameter T2 ist dann die Zeit bis zum Erreichen des 0,1fachen Teil des Sollwertes S1 und die Summe von T1 und T2 die Zeit bis zum 0,8fachen Teil des Sollwerts S1. Die Systemparameter T1 und T2 beschreiben den momentanen Zustand des Atemkreislaufs (1) und sind abhängig beispielsweise von dem durch die Gasdosiereinheit (2) in den Atemkreislauf (1) dosierten Anästhesiegasfluß und den am Ventilator (101) eingestellten Beatmungsparametern wie Atemfrequenz f und Atemhubvolumen Vr.

Hat die Anästhesiemittelkonzentration den 0,9fachen Teil des Sollwertes S1 erreicht, und damit den Punkt S3, schaltet der Trennschalter (311) in Schließrichtung und der Anästhesiemittel-Regler (302) beeinflußt die Anästhesiemittel-Dosierpumpe (307) zur Einstellung des neuen Sollwertes S1 im Atemkreislauf (1).

Aus den gemessenen Systemparametern T1 und T2 werden in der Anpaßschaltung (305) Einstellparameter (k, $C_I$, $C_D$) für den Anästhesiemittel-Regler (302) errechnet und diesem eingeprägt. Hierbei ist es zweckmäßig, Teile der Berechung in der Steuereinheit (5) durchzuführen und die Signale über eine Steuerleitung (866) an die Anpaßschaltung (305) zu übertragen. Weiter ist vorstellbar, daß Parameter des Patienten in die Steuereinheit eingegeben und bei der Berechnung der Einstellparameter berücksichtigt werden.

Die formelmäßigen Zusammenhänge zwischen den Systemparametern T1 und T2 und den Einstellparametern (k, $C_I$, $C_D$) des Anästhesiemittel-Reglers (302) sind in Fig. 3 angegeben. Die Zeitkonstante $T_0$ ist hierbei die reziproke Atemfrequenz f. Diese Berechnungsformeln basieren auf den Optimierungskriterien von Ziegler und Nichols (Optimum Settings for Automatic Controllers, Transactions of the A.S.M.E, November 1942).

## Ansprüche

1. Verfahren zum Betreiben eines Anästhesiebeatmungsgerätes mit
- einer in zwei Zweige aufgeteilten Atemkreisführung,
- einem $CO_2$-Absorber in einem der Zweige,
- einer Dosiervorrichtung für Anästhesiegase,
- einer Meßeinrichtung zur Analyse der Anästhesiegase,
- mehreren koppelbaren Anästhesiegas-Regelkreisen,
- einer Steuervorrichtung für die Dosiervorrichtung, Meßeinrichtung und Anästhesiegas-Regelkreise,
dadurch gekennzeichnet, daß mindestens ein Anästhesiegas-Regelkreis mit einem Trennschalter (311) versehen ist, und daß zur Einstellung eines neuen Anästhesiegas-Sollwertes S1 die Steuereinheit (5)
- den Trennschalter (311) während einer vorbestimmten Zeitdauer in Öffnungsstellung schaltet,
- die Anästhesiegas-Stellgröße an der Dosiereinheit (307) sprunghaft auf einen vom Sollwert S1 abweichenden Anflutungssollwert S2 einstellt,
- aus dem mit der Meßeinrichtung (106) in mindestens einem der Zweige (113,114) gemessenen zeitlichen Verlauf der zugehörigen Anästhesiegaskonzentration Atemkreis-Systemparameter T1 und T2 bestimmt und hieraus zumindest Einstellparameter (k, $C_I$, $C_D$) für den zugehörigen Anästhesiegas-Regler (302) errechnet,
- und daß die Steuereinheit (5) bei Erreichen des Anästhesiegaskonzentrationswertes S3 den Trennschalter (311) in Schließstellung schaltet, wodurch der Anästhesiegas-Regler (302) die Dosiereinheit (307) zur Einstellung des Sollwertes S1 beeinflußt.

2. Verfahren nach Anspruch 1, dadurch gekennzeichnet, daß der Anästhesiegas-Sollwert die Anästhesiemittelkonzentration (814) ist.

3. Verfahren nach Anspruch 1, dadurch gekennzeichnet, daß der Anästhesiegas-Sollwert die Sauerstoffkonzentration (813) ist.

4. Verfahren nach Anspruch 1, dadurch gekennzeichnet, daß der Anästhesiegas-Sollwert die Anästhesie-Ventilation in Form der Kohlendioxid-Konzentration (815) ist.

5. Verfahren nach Anspruch 1 bis 4, dadurch gekennzeichnet, daß der Konzentrationswert S2 bei einer Konzentrationserhöhung bis auf das 10fache vom Sollwert S1 eingestellt, bei einer Konzentrationserniedrigung auf Null reduziert wird.

6. Verfahren nach einem der Ansprüche 1 bis 5, dadurch gekennzeichnet, daß der Konzentrationswert S3 bei einer Konzentrationserhöhung auf das 0,9fache des Sollwertes S1, bei einer Konzentrationserniedrigung auf das 1,1fache des Sollwertes S11 eingestellt wird.

7. Verfahren nach einem der Ansprüche 1 bis 6, dadurch gekennzeichnet, daß als Systemparameter T2 bei einer Konzentrationserhöhung die Zeit bis zum Erreichen des Konzentrationswertes vom 0,1 fachen vom Sollwert S1, und als die Summe der Parameter T1 und T2 die Zeit bis zum Erreichen des Konzentrationswertes vom 0,8fachen des Sollwertes S1 vorgesehen ist.

8. Verfahren nach einem der Ansprüche 1 bis 7, dadurch gekennzeichnet, daß Regelkreise mit Reglern für die Anästhesiegasbestandteile Sauerstoffkonzentration (206), Anästhesiemittelkonzentration (302), Atemkreisvolumen (222) und Kohlendioxid-Konzentration (402) zwischen Atemkreislauf (1) und Steuereinheit (5) angeschlossen sind und mindestens zwei dieser Regelkreise über Koppelglieder (224,405,205,304,305) verbunden sind.

9. Verfahren nach einem der Ansprüche 1 bis 8, dadurch gekennzeichnet, daß einzelne Koppelglieder (224, 405, 205, 304, 305) und einzelne Regler (302, 206, 402) über Signalleitungen mit der Steuereinheit (5) verbunden sind und von dieser angesteuert werden.

10. Verfahren nach einem der Ansprüche 1 bis 9, dadurch gekennzeichnet, daß die Reihenfolge der Sollwertvorgaben (813, 814, 815) durch die Steuereinheit (5) festgelegt wird.

11. Verfahren nach einem der Ansprüche 1 bis 10, dadurch gekennzeichnet, daß die Reihenfolge der Sollwertvorgaben in Sauerstoffkonzentrationen (813), Anästhesiemittelkonzentration (814) und Kohlendioxid-Konzentration (815) besteht.

FIG. 1

EP 0 397 011 A2

FIG. 2

$$k = \frac{1,2 \cdot T_1}{(T_2 + T_0)} - \frac{0,3 \cdot T_1 \cdot T_0}{(T_2 + T_0/2)^2} \; ; \; T_0 = \frac{1}{f}$$

$$C_I = \frac{0,6 \cdot T_1 \cdot T_0}{k \, (T_2 + T_0/2)^2}$$

$$C_D = \frac{0,5 \cdot T_1}{k \quad T_0}$$

**FIG. 3**

EP 0 397 011 A2